(19) Europäisches Patentamt
European Patent Office
Office européen des brevets

(11) EP 4 649 949 A1

(12) # EUROPEAN PATENT APPLICATION
published in accordance with Art. 153(4) EPC

(43) Date of publication:
**19.11.2025 Bulletin 2025/47**

(21) Application number: **24741548.2**

(22) Date of filing: **11.01.2024**

(51) International Patent Classification (IPC):
***A61K 31/7088*** (2006.01) ***A61K 31/712*** (2006.01)
***A61K 31/7125*** (2006.01) ***A61K 47/60*** (2017.01)
***A61P 27/02*** (2006.01) ***C12N 15/115*** (2010.01)

(52) Cooperative Patent Classification (CPC):
**A61K 31/7088; A61K 31/712; A61K 31/7125; A61K 47/60; A61K 48/00; A61P 27/02; C12N 15/115**

(86) International application number:
**PCT/JP2024/000339**

(87) International publication number:
**WO 2024/150776 (18.07.2024 Gazette 2024/29)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **12.01.2023 JP 2023003260**

(71) Applicants:
- **RIBOMIC INC.**
  **Minato-ku**
  **Tokyo 108-0071 (JP)**
- **Nihon University**
  **Tokyo 102-8275 (JP)**
- **National University Corporation**
  **Asahikawa Medical University**
  **Asahikawa, Hokkaido 078-8510 (JP)**

(72) Inventors:
- **NAGAOKA, Taiji**
  **Asahikawa-shi, Hokkaido 078-8510 (JP)**
- **HANAZAKI, Hirotsugu**
  **Tokyo 102-8275 (JP)**
- **NAKAMURA, Yoshikazu**
  **Tokyo 108-0071 (JP)**

(74) Representative: **Engelhard, Markus**
**Boehmert & Boehmert**
**Anwaltspartnerschaft mbB**
**Pettenkoferstrasse 22**
**80336 München (DE)**

Remarks:
The complete document including Reference Table(s) and the Sequence Listing(s) can be downloaded from the EPO website

(54) **PHARMACEUTICAL COMPOSITION FOR PREVENTING PROLIFERATIVE VITREORETINOPATHY CONTAINING AUTOTAXIN APTAMER**

(57) A problem of the present invention is to provide a novel pharmaceutical composition for preventing proliferative vitreoretinopathy. The present invention solves the above-mentioned problem by providing a pharmaceutical composition for preventing proliferative vitreoretinopathy, containing an autotaxin aptamer or a salt thereof.

EP 4 649 949 A1

## Description

[Technical Field]

The present invention relates to a pharmaceutical composition for preventing proliferative vitreoretinopathy, containing an autotaxin aptamer, and the like.

[Background Art]

It is known that proliferative vitreoretinopathy (PVR) is developed due to rhegmatogenous retinal detachment (RRD) or trauma, and once developed, it becomes an extremely intractable disease. The progression from RRD to PVR is about 10%, and various cells and cytokines are said to be involved in the development of PVR. Among them, retinal pigment epithelial (RPE) cells are said to be the main cells involved in the development of PVR. The vitreous body is a transparent, jelly-like tissue that fills the lumen of the eyeball behind the crystalline lens (corresponding to the lens of a camera). When a retinal tear occurs and RRD develops, RPE cells disperse in the vitreous body and attach to the retinal surface (corresponding to the film of a camera). Along therewith, epithelial-mesenchymal transition (EMT) of RPE cells occurs to change them to fibroblast-like cells, thus resulting in fibrosis. The interaction between fibrotic RPE cells and the extracellular matrix forms a proliferative membrane and causes retinal traction contraction, which is the cause of PVR. Various cytokines and growth factors are said to be involved in the process of PVR development, but the detailed mechanism is still unknown.

The main treatment method for PVR is surgery, but severe visual impairment remains even after surgery as the prognosis of PVR. As a non-surgical adjuvant therapy, drug treatments have been considered, and, for example, corticosteroid, dasatinib, resveratrol, and the like have been reported, but the therapeutic effects thereof vary.

Autotaxin (ATX) is an enzyme that produces lysophosphatidic acid (LPA) and is widely distributed in the circulating blood, cerebrospinal fluid, kidneys, and lymphatic organs. The ATX enzyme, lysophospholipase D, converts lysophosphatidylcholine (LPC) to LPA. LPA is one of the lysophospholipids, which are lipid mediators. After conversion of LPA from LPC, LPA is released outside the cell and binds to extracellular receptors to show physiological activity. After binding to an extracellular receptor, LPA activates downstream pathways, thereby exhibiting actions such as cell proliferation, migration, intercellular adhesion, fibrosis, and the like.

There is a recent report on the growth-suppressing effect of ATX inhibition in pulmonary fibrosis model mice using an aptamer that binds to autotaxin (hereinafter also to be referred to as "autotaxin aptamer") (Patent Literature 1). Aptamers have attracted attention as molecular targeting substances that can replace antibodies, and have advantages such as stronger target binding power than antibodies, no restrictions on the target, ease of chemical modification, and low antigenicity. However, the relationship between ATX and PVR has not been reported in detail to date.

[Citation List]

[Patent Literature]

[Patent Literature 1]
WO 2015/147290

[Summary of Invention]

[Technical Problem]

An object of the present invention is to provide a novel pharmaceutical composition and the like for preventing PVR.

[Solution to Problem]

The present inventors investigated diligently to solve the problem described above and found that a high-quality aptamer against autotaxin prevents PVR, which resulted in the completion of the present invention.

Accordingly, the present invention provides the following invention.

[1] A pharmaceutical composition for preventing proliferative vitreoretinopathy, comprising an autotaxin aptamer or a salt thereof.

[2] The pharmaceutical composition of [1], wherein the autotaxin aptamer comprises a nucleotide sequence

represented by the following formula: GWAACAGGUUUUGCU (SEQ ID NO: 1) wherein W is A or U (provided that uracil is optionally thymine), has a length of 21 nucleotides or more, and is either the following (a) or (b):

(a) an aptamer wherein, in the nucleotides contained in the aptamer,

(i) the 2'-position of ribose of each pyrimidine nucleotide is a fluorine atom,
(ii) the 2'-position of ribose of each purine nucleotide is a hydroxy group;

(b) the aptamer of (a), wherein

(i) the fluorine atom at the 2'-position of the ribose of each pyrimidine nucleotide is independently unsubstituted, or substituted by an atom or group selected from the group consisting of a hydrogen atom, a hydroxy group and a methoxy group,
(ii) the hydroxy group at the 2'-position of the ribose of each purine nucleotide is independently unsubstituted, or substituted by an atom or group selected from the group consisting of a hydrogen atom, a methoxy group and a fluorine atom.

[3] The pharmaceutical composition of [1], wherein the autotaxin aptamer comprises a nucleotide sequence represented by the following formula:
UCUGAGGGAAACAGGUUUUGCUCCUCGGA (SEQ ID NO: 2)
(provided that uracil is optionally thymine), and is either the following (a) or (b):

(a) an aptamer wherein, in the nucleotides contained in the aptamer,

(i) the 2'-position of ribose of each pyrimidine nucleotide is a fluorine atom,
(ii) the 2'-position of ribose of each purine nucleotide is a hydroxy group;

(b) the aptamer of (a), wherein

(i) the fluorine atom at the 2'-position of the ribose of each pyrimidine nucleotide is independently unsubstituted, or substituted by an atom or group selected from the group consisting of a hydrogen atom, a hydroxy group and a methoxy group,
(ii) the hydroxy group at the 2'-position of the ribose of each purine nucleotide is independently unsubstituted, or substituted by an atom or group selected from the group consisting of a hydrogen atom, a methoxy group and a fluorine atom.

[4] The pharmaceutical composition of [2] or [3], wherein at least one nucleotide contained in the autotaxin aptamer is modified or altered.
[5] The pharmaceutical composition of [2] or [3], wherein at least one nucleotide contained in the autotaxin aptamer is modified inverted dT or polyethylene glycol.
[6] The pharmaceutical composition of [2] or [3], wherein inverted dT or polyethylene glycol is bound to the 5' end or 3' end of the autotaxin aptamer.
[7] The pharmaceutical composition of [2] or [3], wherein at least one phosphate group contained in the autotaxin aptamer is phosphorothioated or phosphorodithioated.
[8] The pharmaceutical composition of [1], wherein the autotaxin aptamer consists of the nucleotide sequence shown by the following formula:

GL2-400GS2-Y-
tC(M)U(M)G(M)A(M)G(M)G(M)gA(M)AsA(M)C(F)A(M)ggU(F)U(F)U(F)U(F)G(M)C(F)U(M)cC(M)U(M)cG(M)G(M)A(M)-idT

wherein each of A, G, U and C is a ribonucleotide, each of g, c, and t is a deoxyribonucleotide, (M) to the right of each ribonucleotide is a methoxy group at the 2' position of ribose, (F) to the right of each ribonucleotide is a fluorine atom at the 2' position of ribose, GL2-400GS2 is SUNBRIGHT (registered trademark) GL2-400GS2, idT is inverted-dT, Y is an ssH linker, and s to the right of A indicates phosphorothioation of a phosphate group of the A.
[9] The pharmaceutical composition of [1], wherein the proliferative vitreoretinopathy is proliferative vitreoretinopathy

that occurs after surgery for rhegmatogenous retinal detachment.

[10] A method for preventing proliferative vitreoretinopathy, comprising a step of administering an autotaxin aptamer or a salt thereof to a subject.

[11] The method of [10], wherein the proliferative vitreoretinopathy is proliferative vitreoretinopathy that occurs after surgery for rhegmatogenous retinal detachment.

[12] An autotaxin aptamer or a salt thereof for use in preventing proliferative vitreoretinopathy.

[13] The autotaxin aptamer or a salt thereof of [12], wherein the proliferative vitreoretinopathy is proliferative vitreoretinopathy that occurs after surgery for rhegmatogenous retinal detachment.

[14] Use of an autotaxin aptamer or a salt thereof in the production of a pharmaceutical composition for preventing proliferative vitreoretinopathy.

[15] The use of [14], wherein the proliferative vitreoretinopathy is proliferative vitreoretinopathy that occurs after surgery for rhegmatogenous retinal detachment.

[Advantageous Effects of Invention]

A high-quality aptamer against autotaxin can be useful as a medicine for preventing PVR.

[Brief Description of Drawings]

[Fig. 1]

A diagram showing the in vitro suppressive effect of an autotaxin aptamer on RPE cell proliferation. **P<0.01

[Fig. 2]

A diagram showing in vitro suppressive effect of an autotaxin aptamer on RPE cell migration. (A) A diagram showing the trace of cell migration within a defined zone (white dotted circle). (B) A graph showing the area of RPE cell migration. **P<0.01

A diagram showing the in vitro suppressive effect of an autotaxin aptamer on RPE cell proliferation. (A) Trace of cell migration within a defined zone (white dotted circle). (B) Graph showing the area of RPE cell migration. **P<0.01

[Fig. 3]

A diagram showing the in vivo effect of an autotaxin aptamer on PVR. (A) Administration of an autotaxin aptamer inhibited the development of tractional retinal detachment (TRD) in PVR. TRD occurred in the control group not administered with an autotaxin aptamer. (B) Administration of an autotaxin aptamer significantly suppressed the development of TRD in PVR compared to non-administration. *P<0.05

[Fig. 4]

A diagram showing the histological image of pig retina after administration of an autotaxin aptamer. Bar: 50 μm

[Description of Embodiments]

<Pharmaceutical composition of the present invention>

The present invention provides a pharmaceutical composition for preventing proliferative vitreoretinopathy, containing an autotaxin aptamer (hereinafter also to be referred to as the aptamer of the present invention) or a salt thereof (hereinafter also to be referred to as the pharmaceutical composition of the present invention).

In the present specification, the "aptamer" refers to a nucleic acid molecule having a binding activity to a particular target molecule. The aptamer can inhibit the activity of a particular target molecule by binding to the particular target molecule. The aptamer of the present invention has a binding activity to autotaxin, and can inhibit the activity of autotaxin. The aptamer of the present invention may be a DNA, an RNA, a modified nucleic acid or a mixture thereof. The aptamer of the present invention can also be in a linear or circular form.

"Autotaxin" (EC.3.1.4.39) is a glycoprotein present in the blood, and is an enzyme that degrades lysophosphatidylcholine (LPC) into lysophosphatidic acid (LPA) and choline. The aptamer of the present invention can exhibit an inhibitory activity against autotaxin derived from any mammals. Such mammals include primates (e.g., human, monkey), rodents (e.g., mouse, rat, guinea pig, hamster), and companion animals, domestic animals and working animals (e.g., dog, cat, horse, bovine, goat, sheep, swine), preferably human.

As for human autotaxin, 4 isotypes of α, β, γ, δ have been reported. In the present invention, human autotaxin particularly means β type. The amino acid sequence of human β-autotaxin is identified by NCBI accession number NP_001035181, and the human autotaxin also includes a partial protein having a substantially equivalent LPA synthesis activity and a mutated protein wherein a part of the amino acid is substituted, deleted, added or inserted.

The aptamer of the present invention binds to autotaxin in a physiological buffer. While the buffer is not particularly limited, one having pH about 5.0 - 10.0 is preferably used. Examples of such buffer include acetate buffer, phosphate buffer, citrate buffer, boric acid buffer, tartrate buffer, Tris buffer, and the like. The aptamer of the present invention binds to autotaxin with the strength of a level detectable by any test shown below.

Biacore T100 manufactured by GE Healthcare is used for the measurement of binding strength of the aptamer of the present invention to autotaxin. In one measurement method, an aptamer is first immobilized on a sensorchip. The immobilization amount is set to about 1500RU. An autotaxin solution as an analyte prepared to 0.020 μM is injected by 20 μL, and binding of the autotaxin to the aptamer is detected. Using RNA containing a random nucleotide sequence consisting of 30 nucleotides as a negative control, when the autotaxin significantly strongly bound to the aptamer as compared to the control RNA, the aptamer can be judged to have a binding ability to autotaxin.

In another measurement method, autotaxin is first immobilized on a sensorchip. The immobilization amount of autotaxin is set to about 2700RU. An aptamer solution as an analyte prepared to 0.30 μM is injected by 20 μL, and binding of the aptamer to the autotaxin is detected. Using RNA containing a random nucleotide sequence consisting of 30 nucleotides as a negative control, when the aptamer significantly strongly bonded to the autotaxin as compared to the control RNA, the aptamer can be judged to have a binding ability to autotaxin.

The inhibitory activity against autotaxin means an inhibitory ability against any activity that the autotaxin has. For example, while autotaxin has a phosphodiesterase activity to cleave phosphodiester bond by hydrolysis, such activity is inhibited. An acceptable substrate for enzyme activity is not limited to a phosphodiester bond-containing substance (e.g., ATP and the like) present in vivo, and includes a substrate wherein a compound containing same is added with a chromogenic substance or a fluorescent substance. The chromogenic substance and fluorescent substance are known to those of ordinary skill in the art. Also, autotaxin has a lysophospholipase D activity. By this activity, lysophosphatidic acid (LPA) is mainly produced by cleaving the bond on the side opposite from the glycerol backbone of phosphodiester of lysophospholipid. For example, inhibition of autotaxin activity also includes suppression of the production of LPA.

A substrate of autotaxin refers to a substance having a phosphodiester bond to be cleaved by hydrolysis by autotaxin. As a substrate of autotaxin present in vivo, lysophosphatidylcholine (LPC) and sphingosylphosphorylcholine (SPC) are known. The substrate of autotaxin in the present specification also includes LPC and SPC having various carbon chain lengths and degrees of unsaturation, and those added with a chromogenic substance or a fluorescent substance.

Whether an aptamer inhibits the enzyme activity of autotaxin can be evaluated, for example, by the following test. As a substrate of autotaxin, phosphodiester bond-containing synthetic substrate p-nitrophenyl thymidine 5'-monophosphate (pNP-TMP) (SIGMA) is used. A phosphodiester bond is cleaved by hydrolysis, and p-nitrophenol is liberated. The p-nitrophenol develops a yellow color, and the color is detected. For the assay, a 96-well plate (96-Well EIA/RIA Polystyrene Plates, Costar) is used, and the amount of the reaction mixture is 200 μL. Nucleic acid is prepared in a suitable buffer (100 μL), 10 mM pNP-TMP (20 μL) adjusted in a buffer in the same manner is added, and the mixture is stirred well and heated at 37°C for 5 min. On the other hand, 6 ng of autotaxin (Recombinant Human, R&D) diluted with a buffer is prepared (80 μL), and heated at 37°C for 5 min. After heating, they are mixed to start an enzyme reaction. The final autotaxin concentration in the reaction solution is 0.3 nM, and the final substrate concentration is 1 mM. A plate containing the reaction mixture is heated at 37°C for 24 hr, placed in a microplate reader SpectraMax190 (Molecular Devices) and the absorbance is determined at wavelength 405 nm. The absorbance when nucleic acid is not added as 100% (A0), an enzyme activity rate is determined from the absorbance (A) of each test substance, using the following formula.

[Math. 1]

$$\text{enzyme activity rate} = (A/A0)\text{x}100$$

The concentration ($IC_{50}$) of an inhibitor necessary for inhibiting the enzyme activity by 50% is determined. An aptamer having an $IC_{50}$ value of not more than 0.10 μM is judged to be an aptamer having a superior inhibitory activity (aptamer of good quality).

The aptamer of the present invention is not particularly limited as long as it binds to any part of autotaxin. The aptamer of the present invention is not particularly limited as long as it binds to any part of autotaxin and can inhibit the activity thereof.

The length of the aptamer of the present invention is not particularly limited, and can usually be not more than about 200 nucleotides. For example, it may be not more than about 100 nucleotides, preferably not more than about 50 nucleotides, more preferably not more than about 40 nucleotides, most preferably not more than about 30 nucleotides. When the total number of nucleotides is smaller, chemical synthesis and mass-production will be easier, and there is a major advantage in terms of cost. It is also thought that chemical modification is easy, stability in the body is high, and toxicity is low. The length of the lower limit of the aptamer of the present invention is not particularly limited as long as it includes the common sequence shown in SEQ ID NO: 1 (GWAACAGGUUUUGCU; W is A or U, provided that uracil is

optionally thymine), and the common sequence can have a desired secondary structure (secondary structure represented by the below-mentioned formula (I) or (II)), the aptamer length may be, for example, not less than 21 nucleotides. In a particularly preferred embodiment, the length of the aptamer of the present invention is 21-50 nucleotides, or 21-40 nucleotides.

In one preferred embodiment, the aptamer of the present invention comprises a nucleotide sequence represented by the following formula
GWAACAGGUUUUGCU (SEQ ID NO: 1)
wherein W is A or U (provided that uracil is optionally thymine) (hereinafter to be also referred to as aptamer (I) of the present invention).

The aptamer (I) of the present invention is either the following (a) or (b):

(a) an aptamer (I) of the present invention in which, in the nucleotides contained therein,

(i) the 2'-position of ribose of each pyrimidine nucleotide is a fluorine atom, and
(ii) the 2'-position of ribose of each purine nucleotide is a hydroxy group;

(b) the aptamer of (a), wherein

(i) the fluorine atom at the 2'-position of the ribose of each pyrimidine nucleotide is independently unsubstituted, or substituted by an atom or group selected from the group consisting of a hydrogen atom, a hydroxy group, an alkoxy group (e.g., methoxy group), an acyloxy group (e.g., acetyloxy group) and an amino group (e.g., $-NH_2$ group), preferably a hydrogen atom, a hydroxy group and a methoxy group, and
(ii) the hydroxy group at the 2'-position of the ribose of each purine nucleotide is independently unsubstituted, or substituted by an atom or group selected from the group consisting of a hydrogen atom, a fluorine atom or an alkoxy group (e.g., methoxy group), an acyloxy group (e.g., acetyloxy group) and an amino group (e.g., $-NH_2$ group), preferably a hydrogen atom, a methoxy group and a fluorine atom.

In the formula: GWAACAGGUUUUGCU (SEQ ID NO: 1), W is preferably A.

A sequence represented by the formula: GWAACAGGUUUUGCU (SEQ ID NO: 1) can form a potential secondary structure represented by the formula (I):

[Chem. 1]

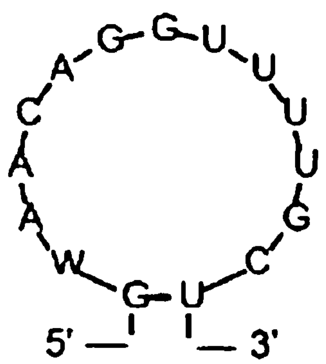

(I)

,

or
when W is A, a potential secondary structure represented by the formula (II):

[Chem. 2]

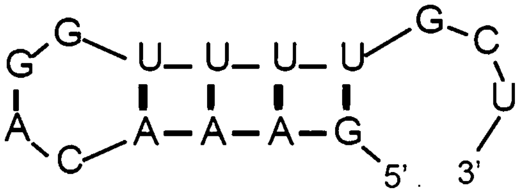
(II)

In aptamer (I) of the present invention, a sequence part of GWAACAGGUUUUGCU (SEQ ID NO: 1) wherein W is A or U can have the above-mentioned structure. Due to this structure, various activities to autotaxin (binding activity to autotaxin, autotaxin inhibitory activity, etc.) are considered to be afforded.

In addition, a stem structure can be preferably formed by an interaction between the sequences following the 5' end and 3' end in the above-mentioned structure. For the aptamer of the present invention to show various activities (binding activity to autotaxin, autotaxin inhibitory activity, etc.), it is desirable to have the above-mentioned potential secondary structure followed by a subsequent stem structure. Having the stem structure, the aptamer is stabilized to have a strong activity. While a stem structure can be formed by complementary base pairs (also including G=U base pairs in addition to Watson-Crick base pairs), the number of base pairs (stem length) is not particularly limited. Preferably, the stem length is not less than 4 base pairs. While the upper limit of the stem length is not particularly limited, for example, it is not more than 18 base pairs, preferably not more than 11 base pairs. In the stem structure, even when base pairs are not formed in a part thereof, the aptamer activity is maintained as long as a stem structure is constituted as a whole. Therefore, the stem structure part can contain substitution, deletion, insertion, or addition of nucleotides as long as the structure thereof is maintained.

In the aptamer (I) of the present invention, 1 to several (e.g., 1 - 4, preferably 1 - 3, more preferably 1 - 2, particularly preferably 1) nucleotides may be substituted, deleted, inserted or added in the nucleotide sequence shown in SEQ ID NO: 1 as long as the binding activity to autotaxin and inhibitory activity against autotaxin activity (enzyme activity of autotaxin etc.) are retained. While the position of substitution, deletion, insertion, or addition of nucleotide is not particularly limited, for example, when a nucleotide is substituted, at least one U of the 10th - 12th UUU from the 5' end of the nucleotide sequence shown in SEQ ID NO: 1 is preferably substituted by other nucleotide, preferably a nucleotide other than A, more preferably C. In the nucleotide sequence shown in SEQ ID NO: 1, when the 6th A from the 5' end is substituted, it is preferably substituted by a nucleotide other than U. More preferably, the 6th A from the 5' end is not substituted.

In the above, the aptamer in which nucleotide(s) is(are) substituted, deleted, inserted or added is either the following (a) or (b):

(a) an aptamer containing the nucleotide sequence shown in SEQ ID NO: 1 in which one to several nucleotides are substituted, deleted, inserted or added,

(i) the 2'-position of ribose of each pyrimidine nucleotide is a fluorine atom, and
(ii) the 2'-position of ribose of each purine nucleotide is a hydroxy group;

(b) the aptamer of (a), wherein

(i) the fluorine atom at the 2'-position of the ribose of each pyrimidine nucleotide is independently unsubstituted, or substituted by an atom or group selected from the group consisting of a hydrogen atom, a hydroxy group, an alkoxy group (e.g., methoxy group), an acyloxy group (e.g., acetyloxy group) and an amino group (e.g., $-NH_2$ group), preferably a hydrogen atom, a hydroxy group and a methoxy group, and
(ii) the hydroxy group at the 2'-position of the ribose of each purine nucleotide is independently unsubstituted, or substituted by an atom or group selected from the group consisting of a hydrogen atom, a fluorine atom or an alkoxy group (e.g., methoxy group), an acyloxy group (e.g., acetyloxy group) and an amino group (e.g., $-NH_2$ group), preferably a hydrogen atom, a methoxy group and a fluorine atom.

Alternatively, in another preferred embodiment, the aptamer of the present invention is an aptamer of any of the following (A) to (C) (hereinafter to be also referred to as the aptamer (II) of the present invention):

(A) an aptamer containing the nucleotide sequence shown in SEQ ID NO: 2 (provided that uracil is optionally thymine);
(B) an aptamer containing the nucleotide sequence shown in SEQ ID NO: 2 (provided that uracil is optionally thymine), wherein 1 - several nucleotides are substituted, deleted, inserted or added nucleotide sequence;
(C) an aptamer containing a nucleotide sequence having identity of not less than 60% (preferably not less than 70%,

more preferably not less than 75%, further preferably not less than 80%, further more preferably not less than 85%, particularly preferably not less than 90%, most preferably not less than 95%) with the nucleotide sequence shown in SEQ ID NO: 2 (provided that uracil is optionally thymine);

(provided that the aptamers of the above-mentioned (B) and (C) can bind to autotaxin to inhibit the activities of autotaxin (enzyme activity etc. of autotaxin)).

The nucleotide sequence shown in SEQ ID NO: 2 of the above-mentioned (B) and (C) contains a sequence represented by the formula: GWAACAGGUUUUGCU (SEQ ID NO: 1) wherein W is A or U) as a common sequence.

In the above-mentioned (B), the number of nucleotides to be substituted, deleted, inserted or added is not particularly limited as long as the aptamer can bind to autotaxin and inhibit activity of autotaxin (enzyme activity etc. of autotaxin). For example, it may be not more than about 10, further preferably not more than 5, most preferably 4, 3, 2 or 1. When the common sequence shown in SEQ ID NO: 1 contains substitution, deletion, insertion or addition of nucleotides, it is preferable to contain mutations similar to those in the above-mentioned aptamer (I) of the present invention. In the nucleotide sequence shown in SEQ ID NO: 1, when the 6th A from the 5' end is substituted, it is preferably substituted by a nucleotide other than U. More preferably, the 6th A from the 5' end is not substituted.

In the above-mentioned (c), the "identity" means a ratio (%) of the same nucleotide residues to all overlapping nucleotide residues in an optimal alignment when two nucleotide sequences are aligned using a mathematical algorithm known in the art (preferably, the algorithm can take into consideration an introduction of a gap into one or both of the sequences for optimal alignment).

In the present specification, the identity of nucleotide sequence can be calculated by, for example, aligning two nucleotide sequences under the following conditions (gap opening =5 penalties; gap extension =2 penalties; x_drop off =50; expectancy =10; filtering=ON) by using homology calculation algorithm NCBI BLAST-2 (National Center for Biotechnology Information Basic Local Alignment Search Tool).

In the above-mentioned aptamer (C), the identity with the common sequence shown in SEQ ID NO: 1 is not less than 80%, preferably not less than 85%, more preferably not less than 90%, and in the nucleotide sequence shown in SEQ ID NO: 1, when the 6th A from the 5' end is substituted, it is preferably substituted by a nucleotide other than U. More preferably, the 6th A from the 5' end is not substituted.

In a particularly preferred embodiment, the aptamer (II) of the present invention is an aptamer of either the following (A'), (B') or (C'):

(A') an aptamer containing the nucleotide sequence shown in SEQ ID NO: 2 (provided that uracil is optionally thymine);
(B') an aptamer containing a nucleotide sequence in which one to several nucleotides are substituted, deleted, inserted or added in the nucleotide sequence shown in SEQ ID NO: 2 (provided that uracil is optionally thymine) in a portion excluding the sequence shown by GAAACAGGUUUUGCU (SEQ ID NO: 3);
(C') an aptamer containing a nucleotide sequence having identity of not less than 70% (preferably not less than 75%, more preferably not less than 80%, further preferably not less than 85%, particularly preferably not less than 90%, most preferably not less than 95%) with the nucleotide sequence shown in SEQ ID NO: 2 (provided that uracil is optionally thymine) (provided that the sequence shown by GAAACAGGUUUUGCU (SEQ ID NO: 3) is the same);
(provided that the aptamers of the above-mentioned (B') and (C') can bind to autotaxin to inhibit the activities of autotaxin (enzyme activity etc. of autotaxin)).

In the above-mentioned (B'), the number of nucleotides to be substituted, deleted, inserted or added is not particularly limited as long as the aptamer can bind to autotaxin and inhibit the activity of autotaxin (enzyme activity of autotaxin, etc.), and may be preferably not more than 5, more preferably 4, further preferably 3, particularly preferably 2 or 1.

In the above-mentioned (C'), "identity" is as defined for the above-mentioned (C).

The aptamer (II) of the present invention can also be
(D) a conjugate of a plurality of one or more of the above-mentioned (A) and/or one or more of the above-mentioned (B) and/or one or more of the above-mentioned (C), preferably (D') a conjugate of a plurality of one or more of the above-mentioned (A') and/or one or more of the above-mentioned (B') and/or one or more of the above-mentioned (C'). In the above-mentioned (D), (D'), conjugation can be achieved by tandem binding. In the conjugation, a linker may be utilized. As the linker, nucleotide chains (e.g., 1 to about 20 nucleotides) and non-nucleotide chains (e.g., $-(CH_2)n-$ linker, $-(CH_2CH_2O)n-$ linker, hexaethylene glycol linker, TEG linker, peptide-containing linker, -S-S- bond-containing linker, -CONH- bond-containing linker, $-OPO_3-$ bond-containing linker) can be mentioned. The plurality as mentioned in the above-described conjugate of a plurality thereof is not particularly limited, as long as it is two or more, and the plurality can be, for example, 2, 3 or 4.

Each aptamer in the above-mentioned (A) - (D), (A') - (D') is either the following (a) or (b):

(a) an aptamer in which, in the nucleotides contained in each aptamer,

(i) the 2'-position of ribose of each pyrimidine nucleotide is a fluorine atom, and
(ii) the 2'-position of ribose of each purine nucleotide is a hydroxy group;

(b) the aptamer of (a), wherein

(i) the fluorine atom at the 2'-position of the ribose of each pyrimidine nucleotide is independently unsubstituted, or substituted by an atom or group selected from the group consisting of a hydrogen atom, a hydroxy group and a methoxy group,
(ii) the hydroxy group at the 2'-position of the ribose of each purine nucleotide is independently unsubstituted, or substituted by an atom or group selected from the group consisting of a hydrogen atom, a methoxy group and a fluorine atom.

The aptamer (II) of the present invention contains, in the above-mentioned (B), (C), (B') or (C'), the nucleotide sequence represented by the formula: GAAACAGGUUUUGCU (SEQ ID NO: 3), and the nucleotide sequence can form a potential secondary structure represented by the formula (I'):

[Chem. 3]

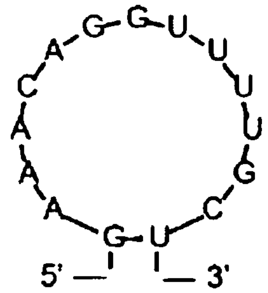

(I')

or the formula (II):

[Chem. 4]

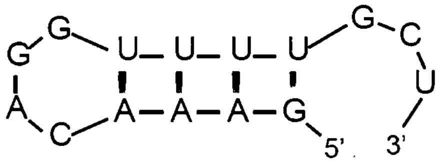

(II)

In addition, the aptamer (II) of the present invention can form a stem structure by an interaction between the sequences following the 5' end and 3' end of the nucleotide sequence represented by the formula: GAAACAGGUUUUGCU (SEQ ID NO: 3) in the above-mentioned (B) or (C). While the stem length is not particularly limited, it is preferably not less than 4 base pairs. Also, the upper limit of the stem length is not particularly limited and, for example, it is not more than 18 base pairs, preferably not more than 11 base pairs. In the stem structure, even when base pairs are not formed in a part thereof, the aptamer activity is maintained as long as a stem structure is constituted as a whole. Therefore, the stem structure part can contain substitution, deletion, insertion, or addition of nucleotides as long as the structure thereof is maintained.

As mentioned above, in the aptamer of the present invention (encompassing the aptamers (I) and (II) of the present invention), at least one kind (e.g., 1, 2, 3 or 4 kinds) of nucleotide can also be a nucleotide having a hydroxyl group, or the above-described any atom or group, for example, at least two kinds (e.g., 2, 3 or 4 kinds) of atoms or groups selected from the group consisting of a hydrogen atom, a fluorine atom, a hydroxyl group and a methoxy group, at the 2'-position of ribose.

In the aptamer of the present invention, all pyrimidine nucleotides may be a nucleotide wherein the 2'-position of ribose is a fluorine atom, or substituted by the aforementioned any atom or group, preferably, the same atom or group selected from the group consisting of a hydrogen atom, a hydroxyl group and a methoxy group.

In the aptamer of the present invention, all purine nucleotides may be a nucleotide wherein the 2'-position of

ribose is a hydroxyl group, or substituted by the aforementioned any atom or group, preferably, the same atom or group selected from the group consisting of a hydrogen atom, a methoxy group and a fluorine atom.

The aptamer of the present invention can also be a nucleotide wherein all nucleotides have the aforementioned any atom or group, for example, the same atom or group selected from the group consisting of a hydrogen atom, a fluorine atom, a hydroxy group and a methoxy group, at the 2'-position of ribose.

In this Description, the nucleotides constituting the aptamer are assumed to be RNAs (i.e., the sugar groups are assumed to be ribose) in describing how the sugar groups are modified in the nucleotides. However, this does not mean that DNA is exempted from the aptamer-constituting nucleotides, and a modification should read as a modification of DNA as appropriate. When the nucleotide constituting the aptamer is DNA, for example, substitution of a hydroxyl group at the 2'-position of ribose by X should read as a substitution of one of the hydrogen atoms at the 2'-position of deoxyribose by X.

The aptamer of the present invention may be one wherein a sugar residue (e.g., ribose) of each nucleotide has been modified to increase the autotaxin binding activity, stability, drug deliverability and the like. As examples of the modification in a sugar residue, substitution of hydroxyl group at the 2'-position, the 3'-position and/or 4'-position of the sugar residue with another atom, and the like can be mentioned. As the kind of the modification, for example, fluorination, alkoxylation (e.g., methoxylation, ethoxylation), O-arylation, S-alkylation (e.g., S-methylation, S-ethylation), S-arylation, and amination (e.g., - $NH_2$) can be mentioned. Such alterations in the sugar residue can be performed by a method known per se (see, for example, Sproat et al., Nucle. Acid. Res., 19, 733-738 (1991); Cotton et al., Nucl. Acid. Res., 19, 2629-2635 (1991); Hobbs et al., Biochemistry, 12, 5138-5145 (1973)).

The sugar residue may also be BNA: Bridged nucleic acid (LNA: Linked nucleic acid), wherein a crosslinking structure is formed at the 2'-position and the 4'-position. Such alteration of the sugar residue can also be performed by a method known per se (see, for example, Obika et al., Tetrahedron Lett., 38, 8735-8738 (1997); Hari et al., Tetrahedron, 59, 5123-5128 (2003); Rahman et al., J. Am. Chem. Soc., 130, 4886-4896 (2008)).

The aptamer of the present invention may also have a nucleic acid base (e.g., purine, pyrimidine) altered (e.g., chemical substitution) to increase the autotaxin binding activity and the like. As examples of such alterations, pyrimidine alteration at 5-position, purine alteration at 6- and/or 8-position(s), alteration with an extracyclic amine, substitution with 4-thiouridine, and substitution with 5-bromo or 5-iodo-uracil can be mentioned.

In addition, the phosphate group contained in the aptamer of the present invention may be altered to confer resistance to nuclease and hydrolysis, or increase the autotaxin binding activity and the like. For example, the P(O)O group as a phosphate group may be substituted by P(O)S (thioate), P(S)S (dithioate), P(O)$NR_2$ (amidate), P(O)R, R(O)OR', CO or $CH_2$ (formacetal) or 3'-amine (-NH-$CH_2$-$CH_2$-) [wherein R and R' are each independently H or a substituted or unsubstituted alkyl (e.g., methyl, ethyl)]. Of the above-mentioned alterations, one wherein at least one of the P(O)O groups to be the phosphate group is substituted by P(O)S (thioate) or P(S)S (dithioate), i.e., phosphorothioated or phosphorodithioated, is preferred. When at least one of the phosphate groups contained in the aptamer is phosphorothioated or phosphorodithioated, the activity of the aptamer of the present invention is improved. While the phosphate group to be phosphorothioated or phosphorodithioated is not particularly limited, for example, it may be in any nucleotide in the common sequence (SEQ ID NO: 1 or 3) in the aptamer (I) or (II) of the present invention, and may be preferably a phosphate group at the third A from the 5' end of the common sequence.

The linking group is, for example, -O-, -N- or -S-, and nucleotides can bind to an adjoining nucleotide via these linking groups.

The aptamer of the present invention may also include alterations such as capping at 3' and 5' of a nucleic acid base.

Terminal modification of the aptamer of the present invention can further be performed by adding, to the 5' end and/or 3' end of the aptamer, polyethyleneglycol, amino acid, peptide, inverted dT, nucleic acid, nucleoside, myristoyl, lithocolic-oleyl, docosanyl, lauroyl, stearoyl, palmitoyl, oleoyl, linoleoyl, other lipid, steroid, cholesterol, caffeine, vitamin, dye, fluorescent substance, anticancer agent, toxin, enzyme, radioactive substance, biotin and the like. As for such terminal modification, see, for example, US Patent 5,660,985 and Kratshmer & Matthew, Nucl. Acid Ther., 27, 335-344 (2017).

The polyethylene glycol to be used for terminal modification of the aptamer may have an average molecular weight of, for example, about 500 Da to about 50 kDa, preferably about 5 kDa to about 40 kDa, more preferably about 10 kDa to about 40 kDa. A polyethylene glycol derivative may also be used to facilitate terminal modification of the aptamer. For example, as described below, a two-chain branched polyethylene glycol derivative such as SUNBRIGHT (registered trademark) GL2-400GS2 can be used. Polyethylene glycol can be linked to the aptamer terminus directly or via a linker. The linker for linking polyethylene glycol to the end of the aptamer is not particularly limited, and for example, an amino linker may be used. For example, as described below, an ssH linker can be used. The ssH linker is an amino linker for chemically modifying the 5' end of a nucleic acid, and the structure thereof is described in Komatsu et al., Bioorg. Med. Chem., 16, 941-949 (2008).

Inverted-dT is a structure that forms a bond between 3'-3' bases at the 3' end of a nucleic acid such as an aptamer and inhibits degradation by 3' exonuclease (Beigelman et al., J. Biol. Chem., 270, 25702-25708 (1995)).

In one particularly preferred embodiment, the aptamer of the present invention is an aptamer consisting of the nucleotide sequence shown by the following formula:

GL2-400GS2-Y-
tC(M)U(M)G(M)A(M)G(M)G(M)gA(M)AsA(M)C(F)A(M)ggU(F)U(F)U(F)U(F)G(M)C(F)U(M)cC
(M)U(M)cG(M)G(M)A(M)-idT

wherein each of A, G, U and C is a ribonucleotide, each of g, c, and t is a deoxyribonucleotide, (M) to the right of each ribonucleotide is a methoxy group at the 2' position of ribose, (F) to the right of each ribonucleotide is a fluorine atom at the 2' position of ribose, GL2-400GS2 is SUNBRIGHT (registered trademark) GL2-400GS2, idT is inverted-dT, Y is an ssH linker, and s to the right of A indicates phosphorothioation of a phosphate group of the A (hereinafter also to be referred to as the aptamer of the present invention (III)).

The aptamer of the present invention can be synthesized as disclosed herein and by a method known per se in the art. One of the synthesis methods is a method using an RNA polymerase. The object RNA can be obtained by chemically synthesizing a DNA having the object sequence and a promoter sequence of RNA polymerase, followed by transcription using same as a template and according to an already-known method. It can be synthesized using DNA polymerase. DNA having an object sequence is chemically synthesized and, using same as a template, amplification is performed by a known method of polymerase chain reaction (PCR). The amplified DNA is converted to a single strand by an already-known method of polyacrylamide electrophoresis or enzyme treatment method. When a modified aptamer is synthesized, the efficiency of elongation reaction can be increased by using a polymerase introduced with a mutation into a specific site. The thus-obtained aptamer can be purified easily by a known method.

Aptamer can be synthesized in a large amount by a chemical synthesis method such as amidite method, phosphoramidite method and the like. The synthesis method is a well-known method, and as described in Nucleic Acid (Vol. 2) [1] Synthesis and Analysis of Nucleic Acid (Editor: Yukio Sugiura, Hirokawa Publishing Company) and the like. In fact, a synthesizer such as OligoPilot plus 100, OligoProcess and the like of GE Healthcare Bioscience can be used. Purification is performed by a method known per se such as chromatography and the like.

A functional substance can be added to the aptamer after synthesis by introducing an active group such as amino group during chemical synthesis by the phosphoramidite method and the like. For example, a polyethylene glycol chain introduced with a carboxyl group, and the like can be condensed by introducing an amino group into the terminal of the aptamer.

An aptamer binds to the target substance in a wide variety of binding modes, such as ionic bond based on the negative charge of the phosphate group, hydrophobic bond and hydrogen bond based on ribose, and hydrogen bond and stacking interaction based on nucleic acid base. In particular, ionic bonds based on the negative charges of the phosphate groups, which are present in the same number as the number of constituent nucleotides, are strong, and bind to the positive charges of lysines and arginines being present on the surface of protein. For this reason, nucleic acid bases not involved in the direct binding to the target substance can be substituted. In particular, because the part of stem structure has already formed base pairs and faces the inside of the double helical structure, nucleic acid bases of the stem structure part are unlikely to bind directly to the target substance. Therefore, even when a base pair is substituted with another base pair, the activity of the aptamer often does not decrease. In structures wherein no base pairs are formed, such as loop structures, provided that the nucleic acid base is not involved in the direct binding to the target molecule, base substitution is possible. Regarding modifications of the 2'-position of ribose, the functional group at the 2'-position of ribose infrequently interacts directly with the target molecule, but in many cases, it is of no relevance, and can be substituted by another modified molecule. Hence, an aptamer, unless the functional group involved in the direct binding to the target molecule is substituted or deleted, often retains the activity thereof. It is also important that the overall steric structure does not change substantially.

An aptamer can be prepared by utilizing SELEX method and an improved method thereof (e.g., Ellington et al., Nature, 346, 818-822 (1990); Tuerk et al., Science, 249, 505-510 (1990)). In the SELEX method, by setting strict selection conditions by increasing the number of rounds or using a competing substance, an aptamer exhibiting a stronger binding potential for the target substance is concentrated and selected. Hence, by adjusting the number of rounds of SELEX and/or changing the competitive condition, aptamers with different binding forces, aptamers with different binding modes, and aptamers with a similar binding force or binding mode but different base sequences can be obtained in some cases. The SELEX method contains a process of amplification by PCR; by causing a mutation by using manganese ions and the like in the process, it is possible to perform SELEX with higher diversity.

The aptamers obtained by SELEX are nucleic acids that exhibit high affinity for the target substance, but this does not mean inhibiting the physiological activity of the target substance. Autotaxin is a basic protein, and is considered to be likely to allow nucleic acids to bind thereto nonspecifically. An aptamer that does not bind to a specific site does not

influence the activity of the target substance.

Based on the active aptamer thus selected, SELEX can be performed using a different primer in an attempt to obtain an aptamer having a higher activity. As a specific method, SELEX is performed again after preparing a template wherein an aptamer with a determined sequence is partially randomized, or a template doped with about 10 to 30% of random sequences.

An aptamer obtained by SELEX has a length of about 80 nucleotides, and this is difficult to prepare as a pharmaceutical as it is. Hence, it is necessary to repeat try-and-error efforts to shorten the aptamer to a length permitting easy chemical synthesis, which is 50 nucleotides or less. Depending on the primer design for an aptamer obtained by SELEX, the ease of the subsequent minimization operation changes. Unless the primer is designed successfully, subsequent development will be difficult even if an aptamer with activity is selected by SELEX.

Aptamers are altered easily since they permit chemical synthesis. For aptamers, by predicting the secondary structure using the MFOLD program, or by predicting the steric structure by X-ray analysis or NMR analysis, it is possible to predict to some extent which nucleotide can be substituted or deleted, where to insert a new nucleotide and the like. A predicted aptamer with the new sequence can easily be chemically synthesized, and it can be determined whether or not the aptamer retains the activity using an existing assay system.

When a part important to the binding of the obtained aptamer with the target substance is identified by repeated try-and-error efforts as described above, the activity remains unchanged in many cases even when a new sequence is added to either end of the sequence. The length of the new sequence is not particularly limited.

Modifications, like sequences, permitting a wide range of design or alterations, can be performed by those of ordinary skill in the art.

The aptamer of the present invention may be in the form of a salt. Examples of the salt include sodium salt, potassium salt, calcium salt, and the like.

In one embodiment, the pharmaceutical composition of the present invention is a pharmaceutical composition for preventing proliferative vitreoretinopathy containing an autotaxin aptamer or a salt thereof, in which the autotaxin aptamer contains a nucleotide sequence represented by the following formula: GWAACAGGUUUUGCU (SEQ ID NO: 1)
wherein W is A or U
(provided that uracil is optionally thymine), has a length of 21 nucleotides or more, and is either the following (a) or (b):

(a) an aptamer wherein, in the nucleotides contained in the aptamer,

(i) the 2'-position of ribose of each pyrimidine nucleotide is a fluorine atom,
(ii) the 2'-position of ribose of each purine nucleotide is a hydroxy group;

(b) the aptamer of (a), wherein

(i) the fluorine atom at the 2'-position of the ribose of each pyrimidine nucleotide is independently unsubstituted, or substituted by an atom or group selected from the group consisting of a hydrogen atom, a hydroxy group and a methoxy group,
(ii) the hydroxy group at the 2'-position of the ribose of each purine nucleotide is independently unsubstituted, or substituted by an atom or group selected from the group consisting of a hydrogen atom, a methoxy group and a fluorine atom.

In one embodiment, the pharmaceutical composition of the present invention is a pharmaceutical composition for preventing proliferative vitreoretinopathy containing an autotaxin aptamer or a salt thereof, in which the autotaxin aptamer contains a nucleotide sequence represented by the following formula: UCUGAGGGAAACAGGUUUUGCUCCUCGGA (SEQ ID NO: 2)
(provided that uracil is optionally thymine), and is either the following (a) or (b):

(a) an aptamer wherein, in the nucleotides contained in the aptamer,

(i) the 2'-position of ribose of each pyrimidine nucleotide is a fluorine atom,
(ii) the 2'-position of ribose of each purine nucleotide is a hydroxy group;

(b) the aptamer of (a), wherein

(i) the fluorine atom at the 2'-position of the ribose of each pyrimidine nucleotide is independently unsubstituted, or substituted by an atom or group selected from the group consisting of a hydrogen atom, a hydroxy group and a methoxy group,

(ii) the hydroxy group at the 2'-position of the ribose of each purine nucleotide is independently unsubstituted, or substituted by an atom or group selected from the group consisting of a hydrogen atom, a methoxy group and a fluorine atom.

In one embodiment, the pharmaceutical composition of the present invention is a pharmaceutical composition for preventing proliferative vitreoretinopathy containing an autotaxin aptamer or a salt thereof, in which the autotaxin aptamer contains a nucleotide sequence represented by the following formula: GWAACAGGUUUUGCU (SEQ ID NO: 1)
wherein W is A or U
(provided that uracil is optionally thymine), contains at least one modified or altered nucleotide, has a length of 21 nucleotides or more, and is either the following (a) or (b):

(a) an aptamer wherein, in the nucleotides contained in the aptamer,

(i) the 2'-position of ribose of each pyrimidine nucleotide is a fluorine atom,
(ii) the 2'-position of ribose of each purine nucleotide is a hydroxy group;

(b) the aptamer of (a), wherein

(i) the fluorine atom at the 2'-position of the ribose of each pyrimidine nucleotide is independently unsubstituted, or substituted by an atom or group selected from the group consisting of a hydrogen atom, a hydroxy group and a methoxy group,
(ii) the hydroxy group at the 2'-position of the ribose of each purine nucleotide is independently unsubstituted, or substituted by an atom or group selected from the group consisting of a hydrogen atom, a methoxy group and a fluorine atom.

In one embodiment, the pharmaceutical composition of the present invention is a pharmaceutical composition for preventing proliferative vitreoretinopathy containing an autotaxin aptamer or a salt thereof, in which the autotaxin aptamer contains a nucleotide sequence represented by the following formula: UCUGAGGGAAACAGGUUUUGCUCCUCGGA (SEQ ID NO: 2)
(provided that uracil is optionally thymine), contains at least one modified or altered nucleotide, and is either the following (a) or (b):

(a) an aptamer wherein, in the nucleotides contained in the aptamer,

(i) the 2'-position of ribose of each pyrimidine nucleotide is a fluorine atom,
(ii) the 2'-position of ribose of each purine nucleotide is a hydroxy group;

(b) the aptamer of (a), wherein

(i) the fluorine atom at the 2'-position of the ribose of each pyrimidine nucleotide is independently unsubstituted, or substituted by an atom or group selected from the group consisting of a hydrogen atom, a hydroxy group and a methoxy group,
(ii) the hydroxy group at the 2'-position of the ribose of each purine nucleotide is independently unsubstituted, or substituted by an atom or group selected from the group consisting of a hydrogen atom, a methoxy group and a fluorine atom.

In one embodiment, the pharmaceutical composition of the present invention is a pharmaceutical composition for preventing proliferative vitreoretinopathy containing an autotaxin aptamer or a salt thereof, in which the autotaxin aptamer contains a nucleotide sequence represented by the following formula: GWAACAGGUUUUGCU (SEQ ID NO: 1)
wherein W is A or U
(provided that uracil is optionally thymine), has a length of 21 nucleotides or more, is modified with inverted dT or polyethylene glycol, and is either the following (a) or (b):

(a) an aptamer wherein, in the nucleotides contained in the aptamer,

(i) the 2'-position of ribose of each pyrimidine nucleotide is a fluorine atom,
(ii) the 2'-position of ribose of each purine nucleotide is a hydroxy group;

(b) the aptamer of (a), wherein

(i) the fluorine atom at the 2'-position of the ribose of each pyrimidine nucleotide is independently unsubstituted, or substituted by an atom or group selected from the group consisting of a hydrogen atom, a hydroxy group and a methoxy group,
(ii) the hydroxy group at the 2'-position of the ribose of each purine nucleotide is independently unsubstituted, or substituted by an atom or group selected from the group consisting of a hydrogen atom, a methoxy group and a fluorine atom.

In one embodiment, the pharmaceutical composition of the present invention is a pharmaceutical composition for preventing proliferative vitreoretinopathy containing an autotaxin aptamer or a salt thereof, in which the autotaxin aptamer contains a nucleotide sequence represented by the following formula: UCUGAGGGAAACAGGUUUUGCUCCUCGGA (SEQ ID NO: 2)
(provided that uracil is optionally thymine), is modified with inverted dT or polyethylene glycol, and is either the following (a) or (b):

(a) an aptamer wherein, in the nucleotides contained in the aptamer,

(i) the 2'-position of ribose of each pyrimidine nucleotide is a fluorine atom,
(ii) the 2'-position of ribose of each purine nucleotide is a hydroxy group;

(b) the aptamer of (a), wherein

(i) the fluorine atom at the 2'-position of the ribose of each pyrimidine nucleotide is independently unsubstituted, or substituted by an atom or group selected from the group consisting of a hydrogen atom, a hydroxy group and a methoxy group,
(ii) the hydroxy group at the 2'-position of the ribose of each purine nucleotide is independently unsubstituted, or substituted by an atom or group selected from the group consisting of a hydrogen atom, a methoxy group and a fluorine atom.

In one embodiment, the pharmaceutical composition of the present invention is a pharmaceutical composition for preventing proliferative vitreoretinopathy containing an autotaxin aptamer or a salt thereof, in which the autotaxin aptamer contains a nucleotide sequence represented by the following formula: GWAACAGGUUUUGCU (SEQ ID NO: 1)
wherein W is A or U
(provided that uracil is optionally thymine), has a length of 21 nucleotides or more, has inverted dT or polyethylene glycol bound to the 5' or 3' end, and is either the following (a) or (b):

(a) an aptamer wherein, in the nucleotides contained in the aptamer,

(i) the 2'-position of ribose of each pyrimidine nucleotide is a fluorine atom,
(ii) the 2'-position of ribose of each purine nucleotide is a hydroxy group;

(b) the aptamer of (a), wherein

(i) the fluorine atom at the 2'-position of the ribose of each pyrimidine nucleotide is independently unsubstituted, or substituted by an atom or group selected from the group consisting of a hydrogen atom, a hydroxy group and a methoxy group,
(ii) the hydroxy group at the 2'-position of the ribose of each purine nucleotide is independently unsubstituted, or substituted by an atom or group selected from the group consisting of a hydrogen atom, a methoxy group and a fluorine atom.

In one embodiment, the pharmaceutical composition of the present invention is a pharmaceutical composition for preventing proliferative vitreoretinopathy containing an autotaxin aptamer or a salt thereof, in which the autotaxin aptamer contains a nucleotide sequence represented by the following formula: UCUGAGGGAAACAGGUUUUGCUCCUCGGA (SEQ ID NO: 2)
(provided that uracil is optionally thymine), has inverted dT or polyethylene glycol bound to the 5' or 3' end, and is either the following (a) or (b):

(a) an aptamer wherein, in the nucleotides contained in the aptamer,

(i) the 2'-position of ribose of each pyrimidine nucleotide is a fluorine atom,
(ii) the 2'-position of ribose of each purine nucleotide is a hydroxy group;

(b) the aptamer of (a), wherein

(i) the fluorine atom at the 2'-position of the ribose of each pyrimidine nucleotide is independently unsubstituted, or substituted by an atom or group selected from the group consisting of a hydrogen atom, a hydroxy group and a methoxy group,
(ii) the hydroxy group at the 2'-position of the ribose of each purine nucleotide is independently unsubstituted, or substituted by an atom or group selected from the group consisting of a hydrogen atom, a methoxy group and a fluorine atom.

In one embodiment, the pharmaceutical composition of the present invention is a pharmaceutical composition for preventing proliferative vitreoretinopathy containing an autotaxin aptamer or a salt thereof, in which the autotaxin aptamer contains a nucleotide sequence represented by the following formula: GWAACAGGUUUUGCU (SEQ ID NO: 1)
wherein W is A or U
(provided that uracil is optionally thymine), has a length of 21 nucleotides or more, contains at least one phosphorothioated or phosphorodithioated phosphate group, and is either the following (a) or (b):

(a) an aptamer wherein, in the nucleotides contained in the aptamer,

(i) the 2'-position of ribose of each pyrimidine nucleotide is a fluorine atom,
(ii) the 2'-position of ribose of each purine nucleotide is a hydroxy group;

(b) the aptamer of (a), wherein

(i) the fluorine atom at the 2'-position of the ribose of each pyrimidine nucleotide is independently unsubstituted, or substituted by an atom or group selected from the group consisting of a hydrogen atom, a hydroxy group and a methoxy group,
(ii) the hydroxy group at the 2'-position of the ribose of each purine nucleotide is independently unsubstituted, or substituted by an atom or group selected from the group consisting of a hydrogen atom, a methoxy group and a fluorine atom.

In one embodiment, the pharmaceutical composition of the present invention is a pharmaceutical composition for preventing proliferative vitreoretinopathy containing an autotaxin aptamer or a salt thereof, in which the autotaxin aptamer contains a nucleotide sequence represented by the following formula: UCUGAGGGAAACAGGUUUUGCUCCUCGGA (SEQ ID NO: 2)
(provided that uracil is optionally thymine), contains at least one phosphorothioated or phosphorodithioated phosphate group, and is either the following (a) or (b):

(a) an aptamer wherein, in the nucleotides contained in the aptamer,

(i) the 2'-position of ribose of each pyrimidine nucleotide is a fluorine atom,
(ii) the 2'-position of ribose of each purine nucleotide is a hydroxy group;

(b) the aptamer of (a), wherein

(i) the fluorine atom at the 2'-position of the ribose of each pyrimidine nucleotide is independently unsubstituted, or substituted by an atom or group selected from the group consisting of a hydrogen atom, a hydroxy group and a methoxy group,
(ii) the hydroxy group at the 2'-position of the ribose of each purine nucleotide is independently unsubstituted, or substituted by an atom or group selected from the group consisting of a hydrogen atom, a methoxy group and a fluorine atom.

In one embodiment, the pharmaceutical composition of the present invention is a pharmaceutical composition for preventing proliferative vitreoretinopathy containing an autotaxin aptamer consisting of the nucleotide sequence shown by

the following formula:

GL2-400GS2-Y-tC(M)U(M)G(M)A(M)G(M)G(M)gA(M)AsA(M)C(F)A(M)ggU(F)U(F)U(F)U(F)G(M)C(F)U(M)cC(M)U(M)cG(M)G(M)A(M)-idT

wherein each of A, G, U and C is a ribonucleotide, each of g, c, and t is a deoxyribonucleotide, (M) to the right of each ribonucleotide is a methoxy group at the 2' position of ribose, (F) to the right of each ribonucleotide is a fluorine atom at the 2' position of ribose, GL2-400GS2 is SUNBRIGHT (registered trademark) GL2-400GS2, idT is inverted-dT, Y is an ssH linker, and s to the right of A indicates phosphorothioation of a phosphate group of the A, or a salt thereof.

As shown in the below-mentioned Examples, the autotaxin aptamer of the present invention suppressed the proliferation and migration of RPE cells in vitro, and reduced the incidence of tractional retinal detachment in proliferative vitreoretinopathy in a swine proliferative vitreoretinopathy model. Therefore, the aptamer of the present invention or a salt thereof can be used as a pharmaceutical composition for preventing proliferative vitreoretinopathy.

The "rhegmatogenous retinal detachment" occurs due to holes or tears in the retina (retinal breaks). In general, retinal detachment often refers to rhegmatogenous retinal detachment. The "proliferative vitreoretinopathy" is a disease in which, when retinal detachment has continued for a long time or when rhegma did not completely close after surgery for rhegmatogenous retinal detachment, fibrous cells increase on the surface of the retina or within the vitreous body in an attempt to heal the wound, due to which the retina is pulled up (in traction) and retinal detachment (tractional retinal detachment) occurs. Therefore, the proliferative vitreoretinopathy prevented by the pharmaceutical composition of the present invention may be proliferative vitreoretinopathy that occurs after surgery for rhegmatogenous retinal detachment.

The pharmaceutical composition of the present invention can be one formulated with a pharmaceutically acceptable carrier. As examples of the pharmaceutically acceptable carrier, excipients such as sucrose, starch, mannit, sorbit, lactose, glucose, cellulose, talc, calcium phosphate, and calcium carbonate; binders such as cellulose, methylcellulose, hydroxypropylcellulose, polypropylpyrrolidone, gelatin, gum arabic, polyethylene glycol, sucrose, and starch; disintegrants such as starch, carboxymethylcellulose, hydroxypropylstarch, sodium-glycol-starch, sodium hydrogen carbonate, calcium phosphate, and calcium citrate; lubricants such as magnesium stearate, Aerosil, talc, and sodium lauryl sulfate; flavoring agents such as citric acid, menthol, glycyllycine-ammonium salt, glycine, and orange powder; preservatives such as sodium benzoate, sodium hydrogen sulfite, methylparaben, and propylparaben; stabilizers such as citric acid, sodium citrate, and acetic acid; suspending agents such as methylcellulose, polyvinylpyrrolidone, and aluminum stearate; dispersing agents such as surfactants; diluents such as water, physiological saline, and orange juice; base waxes such as cacao butter, polyethylene glycol, and kerosene; and the like can be mentioned, but these are not limitative.

While the administration route of the pharmaceutical composition of the present invention is not particularly limited, for example, oral administration and parenteral administration can be mentioned. Parenteral administration is preferred.

As preparations suitable for parenteral administration (e.g., intravenous administration, intravitreal administration, subretinal administration, ophthalmic administration, and the like), aqueous and non-aqueous isotonic sterile injectable liquids are available, which may contain an antioxidant, a buffer solution, a bacteriostatic agent, an isotonizing agent and the like. Aqueous and non-aqueous sterile suspensions can also be mentioned, which may contain a suspending agent, a solubilizer, a thickener, a stabilizer, an antiseptic and the like. The preparation can be included in a container such as an ampoule or a vial in a unit dosage volume or in several divided doses. An active ingredient and a pharmaceutically acceptable carrier can also be freeze-dried and stored in a state that may be dissolved or suspended in an appropriate sterile vehicle just before use.

The dosage of the pharmaceutical composition of the present invention varies depending on the kind and activity of active ingredient, seriousness of disease, animal species being the subject of administration, and drug tolerability, body weight, and age of the subject of administration, and the like, and the usual dosage, based on the amount of active ingredient per day for an adult, can be, for example, about 0.0001 to about 100 mg/kg, preferably about 0.0001 to about 10 mg/kg, more preferably about 0.005 to about 1 mg/kg.

<Method for preventing proliferative vitreoretinopathy of the present invention, etc.>

The present invention includes a method for preventing proliferative vitreoretinopathy, including a step of administering an autotaxin aptamer or a salt thereof to a subject; a method for preventing proliferative vitreoretinopathy that occurs after surgery for rhegmatogenous retinal detachment, including a step of administering an autotaxin aptamer or a salt thereof to a subject; an autotaxin aptamer or a salt thereof for use in preventing proliferative vitreoretinopathy; an

autotaxin aptamer or a salt thereof for use in preventing proliferative vitreoretinopathy that occurs after surgery for rhegmatogenous retinal detachment; use of an autotaxin aptamer or a salt thereof in the production of a pharmaceutical composition for preventing proliferative vitreoretinopathy; and use of an autotaxin aptamer or a salt thereof in the production of a pharmaceutical composition for preventing proliferative vitreoretinopathy that occurs after surgery for rhegmatogenous retinal detachment.

The present invention is described in more detail by means of the following Examples, which, however, do not limit the present invention.

[Example]

1. Method and material

1.1. Profile of RBM-006

The nucleotide sequence of RBM-006 is shown below.

40P-Y-tC(M)U(M)G(M)A(M)G(M)G(M)gA(M)AsA(M)C(F)A(M)ggU(F)U(F)U(F)U(F)G(M)C(F)U(M)cC(M)U(M)cG(M)G(M)A(M)-idT

wherein each of A, G, U and C is a ribonucleotide, each of g, c, and t is a deoxyribonucleotide, (M) to the right of each ribonucleotide is a methoxy group at the 2' position of ribose, (F) to the right of each ribonucleotide is a fluorine atom at the 2' position of ribose, GL2-400GS2 is SUNBRIGHT (registered trademark) GL2-400GS2 (NOF CORPORATION), idT is inverted-dT, Y is an ssH linker, and s to the right of A indicates phosphorothioation of a phosphate group of the A.

As described above, RBM-006 is an autotaxin aptamer consisting of 29 nucleotides, the 2' position of ribose of each nucleotide is greatly modified to resist ribonucleases, the 5' and 3' ends thereof are linked to 40 kDa PEG and inverted-dT, respectively, and a sufficient pharmacokinetic profile is obtained (WO 2015/163458). RBM-006 binds stably and specifically to autotaxin and does not bind to other proteins tested, including heparin-binding proteins. Importantly, RBM-006 inhibits conversion activity of autotaxin from LPC to LPA at 0.5 nM ($IC_{50}$ value). The blood half-life of RBM-oo6 is 3.7 hr.

1.2. RPE cell culture

RPE cells were isolated from pig eyes purchased from Tokyo Shibaura Organ Co., Ltd. (Tokyo, Japan). Primary culture of RPE cells followed an established protocol (Sonoda et al., Nat. Protoc., 4, 662-673 (2009)). Experiments were performed using 2-6 week-old RPE cells cultured to confluence in 25 $cm^2$ tissue culture flasks, with hexagonal morphology and no visible pigmentation. RPE cells were cultured in DMEM supplemented with glutamine (5 mL), penicillin/streptomycin (5 mL), and 5% fetal bovine serum (FBS) at 37°C and 5% $CO_2$.

1.3. Pig PVR model

The use of animals was approved by the Nihon University Ethics Committee and conformed to the ARVO Statement for the Use of Animals in Ophthalmic and Vision Research (Association for Research in Vision and Ophthalmology; ARVO). PVR was induced in domestic pigs according to a reported protocol (Umazume et al., Invest. Ophthalmol. Vis. Sci., 53, 4910-4916 (2012)). Specifically, vitreous surgery was performed in which three incisions were made, a 25-gauge perfusion circuit, suction and resection cutter, and lighting equipment were inserted from the three incisions, and the peripheral vitreous body was removed. Next, Dulbecco's modified Eagle's medium (high glucose) (Merck, D6171) was injected under the retina using a 39-gauge subretinal injection needle to induce retinal detachment. After completion of the vitreous surgery, RPE cells ($2.0 \times 10^6$ cells) were injected into the eye. Autotaxin aptamer RBM-006 (RIBOMIC Inc.) was suspended in BSS PLUS 500 Intraocular irrigating solution 0.0184% (Alcon Japan Ltd.) to a concentration of 50 mg/ml, and a total of 1 mg of RBM-006 was injected after the RPE cell injection. Furthermore, 1 mg of RBM-006 was injected again on day 7 after surgery, in the same manner as the above. Clinical examinations were performed on days 7 and 14 after surgery. PVR grade was based on a reported protocol (Umazume et al., Invest. Ophthalmol. Vis. Sci., 53, 4910-4916 (2012)).

1.4. Immunofluorescence method and immunohistochemistry method

Since the immunofluorescence method and immunohistochemistry method are described in detail in, for example, the Immunohistochemistry Staining Experiment Handbook (Takara Bio), they are briefly explained here. Samples from domestic pigs were embedded in paraffin blocks and sections thereof were produced. Before starting immunostaining, a deparaffinization treatment was performed, and then antigen activation was performed (110°C, 10 min) using 10 mM citrate buffer (pH 6.0). The sections were washed with PBS (5 min x 3 times of immersion). After washing, the sections were blocked (room temperature, 60 min) using blocking buffer (10% Donkey serum/PBS), and then successively subjected to primary antibody reaction (4°C, overnight), washing with PBS (5 min x 3 times of immersion), secondary antibody reaction (room temperature, 60 min), and washing with PBS (5 min x 3 times of immersion). The antibodies used for immunostaining were anti-SMA antibody (Rabbit) (abcam, ab5694) as the primary antibody and AlexaFluor (registered trademark) 647 anti-Rabbit IgG (H+L) antibody (Donkey) (Invitrogen, A-31573) as the corresponding secondary antibody, anti-Autotaxin monoclonal antibody (Rat) (MBL, D323-3) as the primary antibody and Alexa Fluor (registered trademark) 488 anti-Rat IgG (H+L) antibody (Donkey) (abcam, ab150153) as the corresponding secondary antibody, and anti-Fibronectin (EP5) antibody (mouse) (SantaCruz, SC-8422) as the primary antibody and Alexa Fluor (registered trademark) 546 anti-Mouse IgG (H+L) antibody (Donkey) (Invitrogen, A-10036) as the corresponding secondary antibody. The primary antibodies and secondary antibodies used were each diluted 1:200 and 1:500 in blocking buffer, respectively. Antibodies other than the Anti-Autotaxin monoclonal antibody were used to confirm that the proliferative membrane was derived from EMT (data not shown).

The sections that were immunofluorescently stained were reacted with DAPI staining solution (abcam, ab228549) for 15 min at room temperature, and then washed with PBS (5 min x 3 times of immersion) to stain the nuclei.

The sections were mounted in mounting medium (VECTASHIELD Vibrance Antifade Mounting Medium, Vector Laboratories, H-1700) and observed under a fluorescent microscope.

1.5. Cell proliferation assay

The influence of RBM-006 addition on RPE cell proliferation was analyzed using the Cell Proliferation ELISA, BrdU (colorimetric) kit (Roche Applied Science, 11647229001) and according to the protocol attached to the kit.

1.6. Cell migration assay

Cell migration assay was performed using the Oris Cell Migration Assay kit (Platypus Technologies, CMA1-101) and according to the protocol attached to the kit. The area of cell migration was determined using Photoshop software (Adobe Inc.).

1.7. Statistical analysis

PVR grade was analyzed using the Mann-Whitney U test, and one-way analysis of variance (one-way ANOVA) was used to analyze in vitro assays (cell proliferation assay and cell migration assay). All statistical analyses were performed using EZR software (Jichi Medical University Saitama Medical Center).

2. Results

2.1. Autotaxin aptamer suppresses RPE cell proliferation

RPE cells undergo EMT when dispersed into the vitreous body. EMT of RPE cells is the initial stage of the fibrosis process, which includes cell proliferation, migration, extracellular matrix (ECM) remodeling, and the like in the pathology of PVR. Autotaxin aptamer is considered to inhibit autotaxin and suppress cell proliferation, migration, intercellular adhesion, and fibrosis downstream of the autotaxin-LPA pathway. In this Example, autotaxin aptamer (RBM-006) was added to the medium of RPE cells ($5.0x10^4$ cells) and cell proliferation was examined (liquid volume: 0.1 ml/well). No significant difference was found when 0.005 mg of RBM-006 was added, but the addition of 0.05 mg and 0.5 mg significantly reduced cell proliferation compared to the control without addition of RBM-006, and dose dependency was also observed (Fig. 1).

2.2. Autotaxin aptamer inhibits RPE cell migration

RBM-006 was added to the medium of cultured RPE cells ($5.0x10^4$ cells) (liquid volume: 0.1 ml/well), and cell migration was examined in the same manner as in cell proliferation. No significant difference was found when 0.005 mg and 0.05 mg of RBM-006 were added, but the addition of 0.5 mg of RBM-006 significantly suppressed cell migration

compared to the control without addition of RBM-oo6 (Fig. 2).

2.3. Autotaxin aptamer suppresses progression of PVR in vivo

Next, the inventors used a pig PVR model to examine whether the autotaxin aptamer could be administered in vivo to suppress PVR. Based on the results of in vitro cell proliferation and cell migration experiments, cultured RPE cells ($2.0 \times 10^6$ cells) were injected into the eyes of the pig PVR model after surgery, and 1.0 mg of RBM-006 was administered on day 0 and day 7. Non-administration of the aptamer was used as the control. The grade of PVR was confirmed on day 14, and the incidence of TRD in PVR significantly decreased by administration of RBM-006 compared to non-administration (Fig. 3). When RBM-006 was administered to a total of five eyes, TRD of grade 1 or higher was not observed except for one eye.

In the control, a proliferative membrane was confirmed in the retina by HE (hematoxylin and eosin) staining (black dotted circle). This proliferative membrane was positive for autotaxin (white dotted circle). On the other hand, after administration of RBM-006, no proliferative membrane was observed, and autotaxin-positive tissue was not clearly confirmed (Fig. 4).

[Industrial Applicability]

The pharmaceutical composition of the present invention is useful as a pharmaceutical composition for preventing proliferative vitreoretinopathy . This application is based on a patent application No. 2023-003260 filed in Japan (filing date: January 12, 2023), the contents of which are incorporated in full herein.

## Claims

**1.** A pharmaceutical composition for preventing proliferative vitreoretinopathy, comprising an autotaxin aptamer or a salt thereof.

**2.** The pharmaceutical composition according to claim 1, wherein the autotaxin aptamer comprises a nucleotide sequence represented by the following formula:
GWAACAGGUUUUGCU (SEQ ID NO: 1)
wherein W is A or U
(provided that uracil is optionally thymine), has a length of 21 nucleotides or more, and is either the following (a) or (b):

(a) an aptamer wherein, in the nucleotides contained in the aptamer,

(i) the 2'-position of ribose of each pyrimidine nucleotide is a fluorine atom,
(ii) the 2'-position of ribose of each purine nucleotide is a hydroxy group;

(b) the aptamer of (a), wherein

(i) the fluorine atom at the 2'-position of the ribose of each pyrimidine nucleotide is independently unsubstituted, or substituted by an atom or group selected from the group consisting of a hydrogen atom, a hydroxy group and a methoxy group,
(ii) the hydroxy group at the 2'-position of the ribose of each purine nucleotide is independently unsubstituted, or substituted by an atom or group selected from the group consisting of a hydrogen atom, a methoxy group and a fluorine atom.

**3.** The pharmaceutical composition according to claim 1, wherein the autotaxin aptamer comprises a nucleotide sequence represented by the following formula:
UCUGAGGGAAACAGGUUUUGCUCCUCGGA (SEQ ID NO: 2)
(provided that uracil is optionally thymine), and is either the following (a) or (b):

(a) an aptamer wherein, in the nucleotides contained in the aptamer,

(i) the 2'-position of ribose of each pyrimidine nucleotide is a fluorine atom,
(ii) the 2'-position of ribose of each purine nucleotide is a hydroxy group;

(b) the aptamer of (a), wherein

(i) the fluorine atom at the 2'-position of the ribose of each pyrimidine nucleotide is independently unsubstituted, or substituted by an atom or group selected from the group consisting of a hydrogen atom, a hydroxy group and a methoxy group,
(ii) the hydroxy group at the 2'-position of the ribose of each purine nucleotide is independently unsubstituted, or substituted by an atom or group selected from the group consisting of a hydrogen atom, a methoxy group and a fluorine atom.

4. The pharmaceutical composition according to claim 2 or 3, wherein at least one nucleotide contained in the autotaxin aptamer is modified or altered.

5. The pharmaceutical composition according to claim 2 or 3, wherein at least one nucleotide contained in the autotaxin aptamer is modified inverted dT or polyethylene glycol.

6. The pharmaceutical composition according to claim 2 or 3, wherein inverted dT or polyethylene glycol is bound to the 5' end or 3' end of the autotaxin aptamer.

7. The pharmaceutical composition according to claim 2 or 3, wherein at least one phosphate group contained in the autotaxin aptamer is phosphorothioated or phosphorodithioated.

8. The pharmaceutical composition according to claim 1, wherein the autotaxin aptamer consists of the nucleotide sequence shown by the following formula:

GL2-400GS2-Y-
tC(M)U(M)G(M)A(M)G(M)G(M)gA(M)AsA(M)C(F)A(M)ggU(F)U(F)U(F)U(F)G(M)C(F)U(M
)cC(M)U(M)cG(M)G(M)A(M)-idT

wherein each of A, G, U and C is a ribonucleotide, each of g, c, and t is a deoxyribonucleotide, (M) to the right of each ribonucleotide is a methoxy group at the 2' position of ribose, (F) to the right of each ribonucleotide is a fluorine atom at the 2' position of ribose, GL2-400GS2 is SUNBRIGHT (registered trademark) GL2-400GS2, idT is inverted-dT, Y is an ssH linker, and s to the right of A indicates phosphorothioation of a phosphate group of the A.

9. The pharmaceutical composition according to claim 1, wherein the proliferative vitreoretinopathy is proliferative vitreoretinopathy that occurs after surgery for rhegmatogenous retinal detachment.

10. A method for preventing proliferative vitreoretinopathy, comprising a step of administering an autotaxin aptamer or a salt thereof to a subject.

11. The method according to claim 10, wherein the proliferative vitreoretinopathy is proliferative vitreoretinopathy that occurs after surgery for rhegmatogenous retinal detachment.

12. An autotaxin aptamer or a salt thereof for use in preventing proliferative vitreoretinopathy.

13. The autotaxin aptamer or a salt thereof according to claim 12, wherein the proliferative vitreoretinopathy is proliferative vitreoretinopathy that occurs after surgery for rhegmatogenous retinal detachment.

14. Use of an autotaxin aptamer or a salt thereof in the production of a pharmaceutical composition for preventing proliferative vitreoretinopathy.

15. The use according to claim 14, wherein the proliferative vitreoretinopathy is proliferative vitreoretinopathy that occurs after surgery for rhegmatogenous retinal detachment.

[Fig. 1]

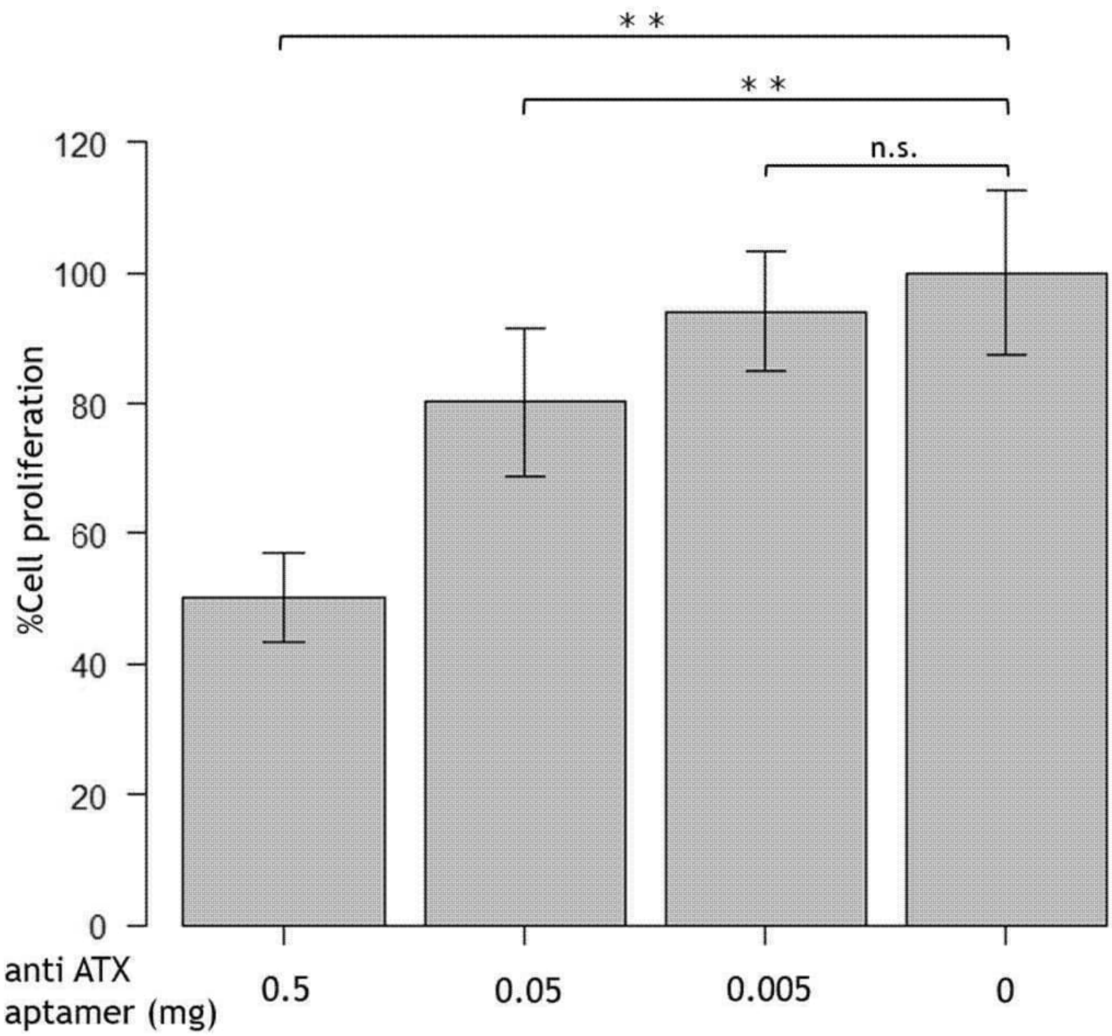

＊＊
＊＊
n.s.
120
100
80
60
40
20
0
%Cell proliferation
anti ATX
aptamer (mg)
0.5
0.05
0.005
0

[Fig. 2]

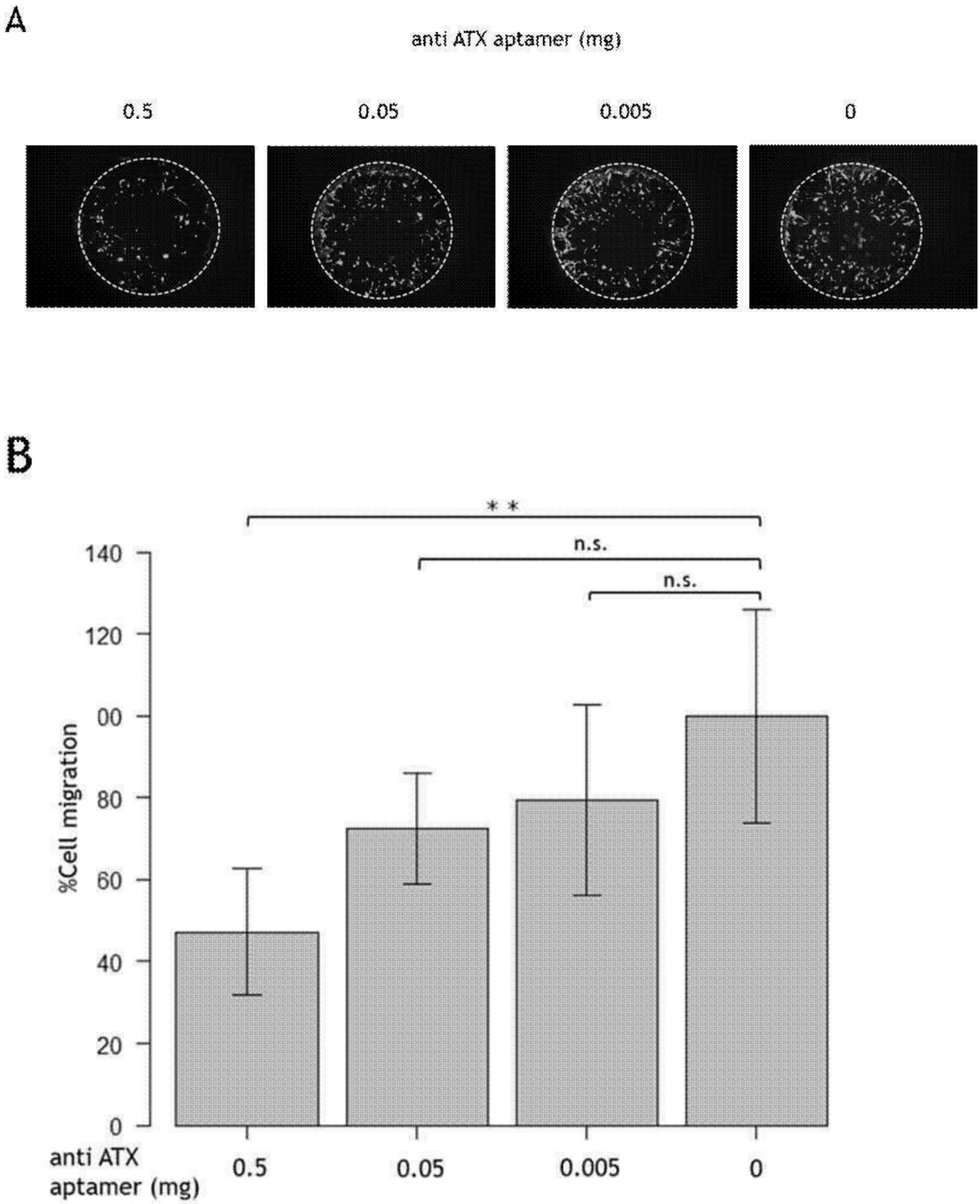
A
anti ATX aptamer (mg)
0.5
0.05
0.005
0
B
* *
n.s.
n.s.
140
120
00
80
60
40
20
0
%Cell migration
anti ATX
aptamer (mg)
0.5
0.05
0.005
0

[Fig. 3]

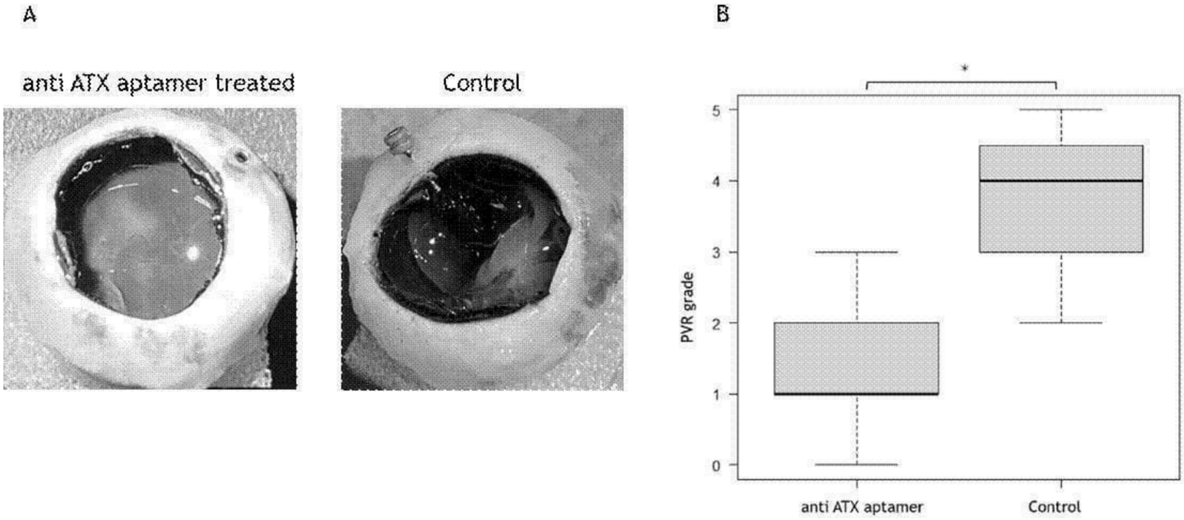
A
anti ATX aptamer treated
Control
B
*
PVR grade
0
1
2
3
4
5
anti ATX aptamer
Control

[Fig. 4]

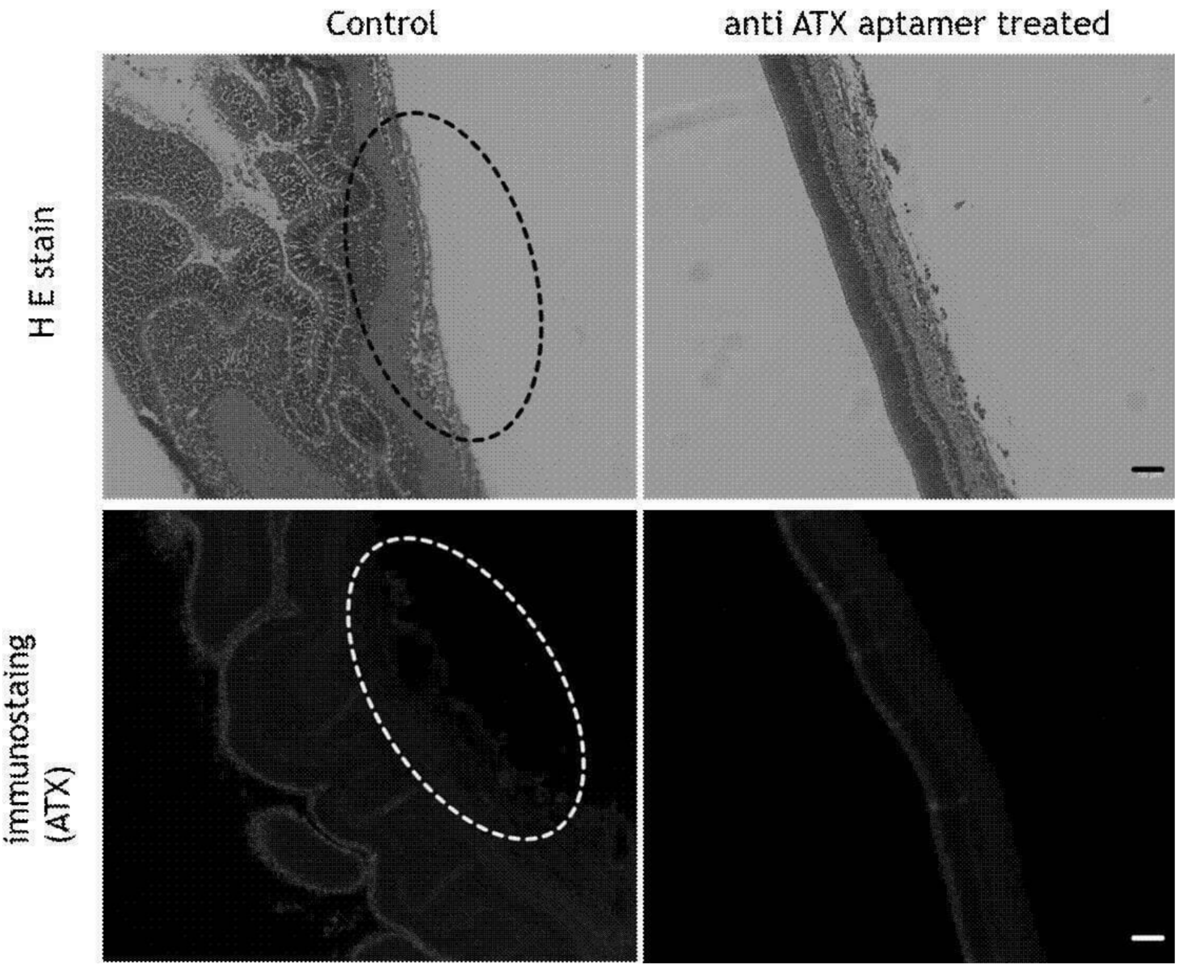
Control
anti ATX aptamer treated
H E stain
immunostaing
(ATX)

**INTERNATIONAL SEARCH REPORT**

International application No. **PCT/JP2024/000339**

**A. CLASSIFICATION OF SUBJECT MATTER**

***A61K 31/7088***(2006.01)i; ***A61K 31/712***(2006.01)i; ***A61K 31/7125***(2006.01)i; ***A61K 47/60***(2017.01)i; ***A61P 27/02***(2006.01)i; ***C12N 15/115***(2010.01)i

FI: A61K31/7088; A61K31/712; A61K31/7125; A61K47/60; A61P27/02; C12N15/115 Z ZNA

According to International Patent Classification (IPC) or to both national classification and IPC

**B. FIELDS SEARCHED**

Minimum documentation searched (classification system followed by classification symbols)

A61K31/7088; A61K31/712; A61K31/7125; A61K47/60; A61P27/02; C12N15/115

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2015/163458 A1 (RIBOMIC INC.) 29 October 2015 (2015-10-29) claim 1 | 12-13 |
| Y | paragraph [0010], claims 1, 15, SEQ ID NO: 16(66) | 1, 9-15 |
| A | | 2–8 |
| X | WO 2015/147290 A1 (RIBOMIC INC.) 01 October 2015 (2015-10-01) claim 1 | 12-13 |
| Y | paragraph [0012], claims 1, 19 | 1, 9-15 |
| A | | 2–8 |
| Y | ABU EL-ASRAR A M et al. Expression of autotaxin and acylglycerol kinase in proliferative vitreoretinal epiretinal membranes. Acta Ophthalmologica. 2012, vol. 90, e 84-e 89 abstract, e87, center column, lines 6-9 | 1, 9-15 |
| A | | 2-8 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 March 2024** | **19 March 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

**INTERNATIONAL SEARCH REPORT**

International application No.
**PCT/JP2024/000339**

**C. DOCUMENTS CONSIDERED TO BE RELEVANT**

| Category* | Citation of document, with indication, where appropriate, of the relevant passages | Relevant to claim No. |
|---|---|---|
| A | AKIYAMA, H. et al. Intraocular Injection of an Aptamer that Binds PDGF-B: A Potential Treatment for Proliferative Retinopathies. Journal of Cellular Physiology. 2006, vol. 207, pp. 407-412 | 1-15 |
| A | 花崎浩継ほか. 裂孔原性網膜剥離における硝子体内のオートタキシン濃度. 第125回日本眼科学会総会講演抄録. 2021, p. 197, O1-109, (HANASAKI, Hirotsugu et al. Concentration of autotaxin in the vitreous of rhegmatogenous retinal detachment), non-official translation (Abstracts of the 125th Annual Meeting of the Japanese Ophthalmological Society.) | 1-15 |
| P, X | HANAZAKI, H. et al. The Effect of Anti-Autotaxin Aptamers on the Development of Proliferative Vitreoretinopathy. International Journal of Molecular Sciences. 03 November 2023, vol. 24, Article.15926<br>abstract, fig. 1-5 | 1-15 |

Form PCT/ISA/210 (second sheet) (July 2022)

**INTERNATIONAL SEARCH REPORT**

International application No.
**PCT/JP2024/000339**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2024/000339**

| Patent document cited in search report | Publication date (day/month/year) | Patent family member(s) | Publication date (day/month/year) |
|---|---|---|---|
| WO 2015/163458 A1 | 29 October 2015 | US 2017/0044545 A1<br>paragraph [0039], claims 1, 15, SEQ ID NO: 16(66)<br>EP 3135766 A1<br>CA 2946709 A<br>AU 2015250584 A<br>CN 106232817 A<br>KR 10-2016-0147884 A | |
| WO 2015/147290 A1 | 01 October 2015 | US 2017/0137818 A1<br>paragraph [0020], claims 1, 19<br>EP 3124607 A1<br>CA 2943788 A<br>AU 2015234724 A<br>CN 106164267 A<br>KR 10-2016-0138215 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015147290 A
- US 5660985 A
- WO 2015163458 A
- JP 2023003260 A


### Non-patent literature cited in the description


- **SPROAT et al.** *Nucle. Acid. Res.*, 1991, vol. 19, 733-738
- **COTTON et al.** *Nucl. Acid. Res.*, 1991, vol. 19, 2629-2635
- **HOBBS et al.** *Biochemistry*, 1973, vol. 12, 5138-5145
- **OBIKA et al.** *Tetrahedron Lett.*, 1997, vol. 38, 8735-8738
- **HARI et al.** *Tetrahedron*, 2003, vol. 59, 5123-5128
- **RAHMAN et al.** *J. Am. Chem. Soc.*, 2008, vol. 130, 4886-4896
- **KRATSHMER** ; **MATTHEW**. *Nucl. Acid Ther.*, 2017, vol. 27, 335-344
- **KOMATSU et al.** *Med. Chem.*, 2008, vol. 16, 941-949
- **BEIGELMAN et al.** *J. Biol. Chem.*, 1995, vol. 270, 25702-25708
- Synthesis and Analysis of Nucleic Acid. Nucleic Acid. Hirokawa Publishing Company, vol. 2
- **ELLINGTON et al.** *Nature*, 1990, vol. 346, 818-822
- **TUERK et al.** *Science*, 1990, vol. 249, 505-510
- **SONODA et al.** *Nat. Protoc.*, 2009, vol. 4, 662-673
- **UMAZUME et al.** *Invest. Ophthalmol. Vis. Sci.*, 2012, vol. 53, 4910-4916